# EUROPÄISCHE PATENTSCHRIFT

(11) **EP 1 863 471 B1**
(45) Veröffentlichungstag und Bekanntmachung des Hinweises auf die Patenterteilung: **15.04.2009**
(21) Anmeldenummer: 06723420.3
(22) Anmeldetag: 14.03.2006
(51) Int. Cl.: A61K 31/22, A61P 29/00

(54) **VERWENDUNG VON ESTERN DER ANGELICASÄURE ALS ANTIPHLOGISTISCHEN WIRKSTOFF, SOWIE ANTIPHLOGISTISCHE ZUBEREITUNGEN**
USE OF ANGELIC ACID ESTERS AS AN ANTIPHLOGISTIC ACTIVE INGREDIENT AND ANTIPHLOGISTIC PREPARATIONS
UTILISATION D'ESTERS DE L'ACIDE ANGELIQUE EN TANT QU'AGENTS ANTIPHLOGISTIQUES, ET PREPARATIONS ANTIPHLOGISTIQUES

(30) Priorität: 15.03.2005 DE 102005012225
(43) Veröffentlichungstag der Anmeldung: 12.12.2007
(73) Patentinhaber: Miltitz Aromatics GmbH, 06766 Wolfen (DE)
(72) Erfinder: GEBAUER, Helmut, 81479 München (DE)
(74) Vertreter: Tragsdorf, Bodo
(86) Internationale Anmeldenummer: PCT/EP2006/002343
(87) Internationale Veröffentlichungsnummer: WO 2006/097283

(56) Entgegenhaltungen:
- CHEN Y-F ET AL: "ANTI-INFLAMMATORY AND ANALGESIC ACTIVITIES FROM ROOTS OF ANGELICA PUBESCENS" PLANTA MEDICA, THIEME, STUTTGART, DE, Bd. 61, Nr. 1, Februar 1995 (1995-02), Seiten 2-8, XP001050391 ISSN: 0032-0943
- WU TIAN-SHUNG ET AL: "Antiinflammatory and analgesic principles from the roots of Angelica pubescens" CHINESE PHARMACEUTICAL JOURNAL, Bd. 46, Nr. 1, 1994, Seiten 45-52, XP009067567 ISSN: 1016-1015
- LIU J H ET AL: "INHIBITORY EFFECTS OF ANGELICA PUBESCENS F. BISERRATA ON 5-LIPOXYGENASE AND CYCLOOXYGENASE" PLANTA MEDICA, THIEME, STUTTGART, DE, Bd. 64, Nr. 6, August 1998 (1998-08), Seiten 525-529, XP009032983 ISSN: 0032-0943
- MELEGARI M ET AL: "CHEMICAL CHARACTERISTICS AND PHARMACOLOGICAL PROPERTIES OF THE ESSENTIAL OILS OF ANTHEMIS-NOBILIS" FITOTERAPIA, Nr. 6, 1988, Seiten 449-456, XP009067566 ISSN: 0367-326X
- ROSSI T ET AL: "SEDATIVE ANTI-INFLAMMATORY AND ANTI-DIURETIC EFFECTS INDUCED IN RATS BY ESSENTIAL OILS OF VARIETIES OF ANTHEMIS-NOBILIS A COMPARATIVE STUDY" PHARMACOLOGICAL RESEARCH COMMUNICATIONS, Bd. 20, Nr. SUPPL. 5, 1988, Seiten 71-74, XP009067563 & IVTH CONGRESS OF THE ITALIAN SOCIETY OF PHARMACOGNOSY, TRIESTE, ITALY, OCTOBER 7-9, 1987. PHARMACOL ISSN: 0031-6989
- CARNAT A ET AL: "The aromatic and polyphenolic composition of Roman camomile tea." FITOTERAPIA, Bd. 75, Nr. 1, Januar 2004 (2004-01), Seiten 32-38, XP002384563 ISSN: 0367-326X in der Anmeldung erwähnt
- MATSUDA HISASHI ET AL: "Inhibitors of nitric oxide production from the flowers of Angelica furcijuga: Structures of hyuganosides IV and V" CHEMICAL & PHARMACEUTICAL BULLETIN (TOKYO), Bd. 53, Nr. 4, April 2005 (2005-04), Seiten 387-392, XP001247090 ISSN: 0009-2363
- GEBAUER, H.: "Pharmacological effects of chamomile active ingredients" SOFW JOURNAL , 132(1/2), 34, 36-38 CODEN: SOFJEE; ISSN: 0942-7694, 2006, XP009067568

## Beschreibung

Die Erfindung betrifft die Verwendung von Estern der Angellcasäure als entzündungshemmendes Mittel, insbesondere für Haut- und Schlelmhautentzündungen, zur Inneren und äußeren Anwendung In Darreichungsformen als Trinkkuren, Kapseln und Aufgüssen, als Zusatz für Sonnenschutzpräparate, Desodorantien, Lippen-, Mund- und Körperpflegemittein, wie Lotions oder After Shaves, Geschirrspülmittein oder Mitteln zur Haar- und Babypflege. Die Verwendung von Extrakten und Ölen sowohl der Deutschen Kamille (Matricarla chamomilla) als auch der Römischen Kamille (Anthemls nobilis) In der Gesundheitsmedizin geht bis In das Altertum zurück. Insbesondere wegen seiner entzündungshemmenden Wirkung zähit die Deutsche Kamille zu den bedeutendsten Hellpflanzen. Extrakt und etherisches Öl werden für äußerliche und innerliche Anwendungen in venschiedensten Darreichungsformen, wie Kapseln, Tees, Bedepräparaten, Cremes, Lotions etc., eingesetzt. Beispiele für Indikationen sind alle Arten von Entzündungen, Ekzeme, Psoriasis, trockene Haut und Sonnenbrand.

Gründlich untersucht wurden bisher die Inhaltsstoffe der Deutschen Kamille. Die antiphlogistische Wirkung wird in erster Linie durch den Gehalt an (-)-Alpha-Bisabolol (bis zu 50%) bestimmt. Andere Substanzen wie Matricin, Chamazulen, Bisabololoxid A, B und C, Bisabolonoxid und Flavonolde, wie Aplgenin und Luteolin, tragen zur Gesamtaktivität bel. Die Im wässrigen Extrakt der Deutschen Kamille enthaltenen Flavonoide tragen zur entzündungshemmenden Wirkung bei, die außerdem als UV-Absorber und Radikalfänger dienen. Aus der Natur gewonnenes (-)-alpha Bisabolol sowie synthetisch hergestelltes racemisches Blsabolol werden heute In bedeutendem Maße In der Gesundheitspflege eingesetzt. Das synthetisch gewonnene Produkt Ist weniger wirksam als die aus dem natürlichen Öl gewonnene minus-Form.

Bekannt Ist auch die Römische Kamille, die aufgrund fehlender Wirksamkeitsnachweise nur volksmedizinisch angewendet wird (Römische Kamille-Chamomillae romanae flos (Ph. Eur. 4. Ausgabe, 3.Nachtrag, Schöpke, www.pharmakobotanlk.de, 2004). Die Inhaltsstoffe der Römischen Kamille, Insbesondere des etherischen Öls, unterscheiden sich von denen der Deutschen Kamille.

Bislang wurden nur einzeine Fraktionen bzw. isollerte Substanzen der Römischen Kamille untersucht. Carnat et al, Fitoterapia 74, 2004, S. 32-38, beschreiben die aromatische und polyphonolische Zusammensetzung von Römischer Kamille, die als entzündungshemmender und spamolytischer Tee bei Magenerkrankungen angewendet wird. Untersucht wurde auch das Öl der Römischen Kamille. Der Gehalt an etherischem Öl der Römischen Kamille Ilegt bei 0,6 bis 2,4 %. Bestandtelle des Öls sind überwlegend Ester der Angelica-, Tiglin-, Methacryl- und Isobuttersäure mit Isobutylalkohol, Isoamylalkohol und 3-Methylamylalkohol sowie weiteren kurzkettigen aliphatischen Alkoholen.

Das etherieche Öl erwles sich als wirksam gegen grampositive Bakterien und Dermatophyten. Antibakterlell wirksam sind die aus dem amanollschen Blütenextrakt Isollerten Hydroperoxide. Nobilin und Nobilinderivate zeigten eine zytostatische Wirkung im invitro-Modellversuch. An Isolierten Polyacetylenen konnten diuretische, zytotoxische und insektizide und an Chammaemelosid blutzuckersenkende Wirkungen festgestellt werden.

Bekannt sind auch kosmetische und paramedizinische Präparate mit Wirkung gegen Entzündungen bzw. hautberuhigender Wirkung, die Insgesamt sieben verschiedene pflanzliche Extrakte enthalten, u.a. Römisches Kamillenöl In sehr geringer Menge, 0,001 bis 0,2 Gew.-%. Aus der EP 04 55 271 B1 sind entzündungshemmende Mittel mit vielen Phorbolverbindungen, darunter zwei Phorbolangelaten, bekannt. Der Diterpene-Alkohol Phorbol ist Bestandtell vieler Wolfsmlich- und Seldelbastgewächse. Der Phorbol-Alkohol Ist die pharmakologisch wirksame Komponente der offenbarten Verbindungen.

Dieses Mittel ist zur Herstellung von Medikamenten zur Behandlung unterschiedlicher Erkrankungen vorgesehen, u.a. auch Entzündungen.

Die DE 44 47 594 A1 offenbart pharmakologisch wirksame Wirkstoffe In enantiomer reiner Form, die aus der Pestwurz gewonnen werden und die nach Verarbeitung zu Arzneimitteln zur Behandlung entzündlicher Erkrankungen eingesetzt werden können. Zu den Wirkstoffen gehören ein Ester der Angelicasäure mit Isopetasin bzw. Petasin. Dabei sind die Wirkstoffe der Pestwurz, nämlich die bicyclischen Alkohole Petasol und Isopetasol, die wirkungsbestimmenden Komponenten.

Bekannt ist die antiphlogistische Wirkung von Extrakten aus Wurzein der Pflanze *Angelica pubencens*, die neben vielen anderen Komponenten Columblanadin enthält (Planta Medica, Thieme Stuttgart, Bd. 61, Nr. 1, Februar 1995, Selten 2-8; Chinese Pharmaceutical Journal, Bd. 46, Nr. 1, 1994, S. 45-52).

Eine entzündungshemmende Wirkung von Ölfraktionen der Römischen Kamille Ist in Fitoterapla, Nr. 6, 1988, S. 449-456, und Pharmacological Research Communications, Bd. 20, Nr. Suppl. 5, 1988, S. 71-74, beschrieben.

Nach wie vor besteht In der Gesundheitspflege, Kosmetik und der Medizin ein Bedarf an entzündungshemmenden Wirkstoffen.

Es Ist Aufgabe der Erfindung, einen weiteren, kostengünstigen Wirkstoff mit antiphlogistischen Eigenschaften bereitzusteilen und ein neues Anwendungsfeld für diesen zu erschließen.

Gelöst wird die Aufgabe durch die erfindungsgemäße Verwendung von den In Anspruch 1 definierten Estern der Angelicasäure als antiphlogistischen Wirkstoff zur Zubereltung von Mittein für Innerliche und äußerliche Anwendungen, sowie die Im Anspruch 6 genannten Ester der Angelicasäure zur Verwendung als antiphlogistischen Wirkstoff zur Zubereitung von Mitteln für innerliche und äußerliche Anwendungen und die antiphlogistische Zubereitung gemäß Anspruch 11.

Zu den erfindungsgemäßen Angelicasäuresstern gemäß den Ansprüchen 1 und 6 gehören Ester der Angelicasäure, die mit Alkoholen, ausgewählt aus Methanol, Ethanol, Propanol, Isopropanol, Butanol, isobutanol, sek. Butanol, Amylalkohol, Isoamylalkohol, Neopentylalkohol, 2-Methylbutanol, 3-Methylbutanol; verzweigten und unverzweigten Hexanolen, Heptanolen, Octanolen, Nonanolen, Decanolen; Allylalkohol, Crotylalkohol, Phenol, Tigilnalkohol, Angelicaalkohol, Sengeloalkohol, trans-2-Hexenol, cls-3-Hexenol, Citroneilol, Geraniol, Laurylalkohol, Cetylalkohol, Nerolidol, Phytol, Ethylenglycol, Propylenglycol, Neopentylglycol, Glycerin und monocyclischen Alkoholen, erhalten werden.

Ausgenommen sind bi-sowie polycyclische Sesquiterpen- und Diterpen-Alkohole. Überraschenderweise wurde gefunden, dass Ester der Angelicasäure bereits mit niederen Alkoholen signifikante entzündungshemmende Elgenschaften sufweisen und sich zur Herstellung von verachledenen Zubereltungen mit antiphlogistischer Wirkung eignen. Es ist überraschend, dass Im Gegensatz zu den kompliziert aufgebauten pharmakologischen Wirkstoffen Bisabolol, Chamazulen der Deutschen Kamille die strukturell einfach aufgebauten Angelicasäureester eine signifikante antiphlogistische Wirkung aufweisen.

Vorteilhafte Ausgestaltungen der Verwendung der Ester der Angelicasäure nach Anspruch 1 sind Gegenstand der Ansprüche 2 bis 6. Vorteilhafte Ausgestaltungen der Ester gemäß Anspruch 6 sind Gegenstand der Ansprüche 7 bis 10.

Zu den erfindungsgemäßen Angellcasäureestern gemäß Anspruch 11 gehören Ester der Angelicasäure, die mit Alkoholen, ausgewählt aus Methanol, Ethanol, Heptenolen, Octanolen, Nonanolen, Decanolen; Allylalkohol, Crotylalkohol, Prenol, Tlglinalkohol, Angelicaalkohol, Senecioalkohol, trans-2-Hexenol, cis-3-Hexanol, Citronellol, Geraniol, Laurylalkohol, Cetylalkohol, Nerolidol oder Phytol, Ethylenglycol, Propylenglycol, Neopentylglycol, Glycerin, Laurylalkohol, Cetylalkohol, Noroildol, Phenol, Benzylalkohol, Phenylothylalkohol und Phenylpropylalkohol, erhalten werden.

Vortellhafte Ausgestaltungen der antiphlogistischen Zubereitung nach Anspruch 11 sind Gegenstand der Ansprüche 12 und 13.

Weder aus der Literatur noch aus der Praxis Ist eine antiphlogistische Wirkung der erfindungsgemäßen Ester der Angellcasäure bekannt.

Zur Anwendung können auch Gemische verschiedener Ester der Angelicasäure kommen. Dies geschieht insbesondere dann, wenn die Duftnote der Zubereitung variiert werden soll oder eine bestimmte Löslichkeit einzustellen ist. Die erfindungsgemäßen Ester werden vorzugsweise synthetisch hergestellt, können aber auch natürlichen Ursprungs sein. Die auf natürlicher Basis beruhenden Ester werden zum Beispiel durch fraktionierte Destillation erhalten.

Die synthetische Herstellung der erfindungsgemäßen Ester erfolgt In an sich bekannter Weise durch Veresterung von Angelicasäure mit den genannten Alkoholen. Die Herstellung ist relativ einfach und kostengünstig. Ein geeignetes Verfahren zur Herstellung von Angelicasäure (cis-2-Methyl-2-butensäure) Ist z.B. in der EP 0 219 601 B1 beschrieben.

Bevorzugt wird als Ester der Angelicasäure Isobutylangelat eingesetzt. Der synthetisch gut zugänglichen Substanz, die bislang lediglich als Riechstoff Verwendung findet, erschließt sich somit ein neues Anwendungsgebiet. Ideal ergänzen sich zudem Duft und Wirkung dieses Esters. Die Angelate besitzen einen warm-würzigen Geruch nach Kamille, Apfel und

Birne. Der psychologische Effekt des kamilleartigen Geruchs unterstützt außerdem noch deren physiologische Aktivität.

Insbesondere Isobutylangelat ist eine wirksame, kostengünstige und zugleich naturidentische Alternative zu den sehr teueren aus der Natur gewonnenen und kompliziert aufgebauten Wirkstoffen. Von Vorteil ist, dass Isobutylangelat eine angenehme und ansprechende Duftnote besitzt und mit relativ geringen Einsatzmengen bereits eine ausreichende Wirkung erzielt wird. Mit Dosierungen von 0,1 bis 0,5 Gew.-% lässt sich eine ausgeprägte entzündungshemmende Wirkung der jeweiligen Zubereltung erzielen, wobei auch höhere oder niedrigere Dosierungen möglich sind.

Zur vorliegenden Erfindung gehören pharmazeutische Zubereitungen, die, neben nichttoxischen, Inerten Trägerstoffen, die erfindungsgemäßen Ester der Angelicasäure enthalten. Bevorzugte Anwendungsformen sind Trinkkuren, Kapseln und Aufgüsse. Außerdem können die Ester als Zusatz für Sonnenschutzpräparate, Desodorantien, Lippen-, Mund- und Körperpflegemittel, Geschirrspülmittel und Mittel zur Haar- und Babypflege eingesetzt werden. Pharmazeutische Zubereitungen dienen sowohl zur Hemmung von entzündlichen Prozessen als auch zur Behandlung akuter Entzündungen.

Pharmazeutische Zubereitungen auf Basis der genannten Angelate werden Innerlich Insbesondere zur Behandlung von Entzündungen des Magen-Darmtraktes sowie äußerlich für Entzündungen aller Art an Haut und Schleimhäuten, bei Beingeschwüren, Ekzemen, Hautreizungen und bei Sonnenbrand eingesetzt.

Kosmetische Zubereitungen sollen keine hautreizenden oder allergisierenden Komponenten enthalten, da sie bei Anwendung auf gereizter und geröteter Haut eine hautberuhigende und entzündungshemmende Wirkung entfaiten sollen. Die erfindungsgemäßen Ester der Angellcasäure werden diesen Anforderungen In vollem Umfang gerecht und erfüllen diese Forderung.

Als Träger für die entzündungshemmenden Angelate kommen alle physlologisch verträglichen Systeme in Frage, in denen die Angelate löslich sind oder sich gleichmäßig dispergieren lassen, wie z.B. Salben, Öl-in-Wasser- oder Wasser-in-Öl-Emulsionen, Mikroemulsionen oder Gele.

Die Angelate können auch in verkapselter Form zur Anwendung gelangen, wie z.B. als "slow release"-System zur verzögerten Frelsetzung des Wirkstoffes. Anwendbare Formen sind z.B. ein mikroverkapselter oder als Cyclodextrin-Komplex molekular eingeschlossener Wirkstoff.

Für topische Anwendungen können auch Stiftpräparate, Sprays oder Puder als Träger dienen.

In Sonnenschutzmitteln werden die Angelate bevorzugt mit Lichtschutzmittein kombiniert. Die vorgenannten Zubereitungen können gegebenenfalls außer den erfindungsgemäßen Verbindungen auch weltere pharmazeutische oder andere Wirkstoffe enthalten.

Die antiinflammatorische Wirkung von Isobutylangelat wurde am Carrageenin-Induzierten (intravenöse Verabreichung) Rattenpfotenödem getestet. Isobutylangelat wurde oral verabreicht.

Die Testbedingungen entsprachen den in der Literatur verwendeten, so dass Quervergleiche hergestellt werden konnten. (V. Jakovlev et al. In Journal of Medicinal Plant Research 35 (1979), 125-140).

Als Vergleichssubstanz In den elgenen Tests diente (-)-alpha-Bisabolol. Dosisabhängig wurde die prozentuale Hemmung der Ödementwichlung bestimmt. Daraus ließ sich der ED₅₀ Wert errechnen.

Doslerungen von 100, 464, 1000 und 2150 mg Isobutylangelat/kg Körpergewicht wurden getestet. Die Ergebnisse wurden mit den für eine Kontrollgruppe (0,8 % Hydroxypropylmethylcellulose Gel) und zusätzlich den für die positive Kontrolle ((-)-alpha- Bisabolol) erhaltenen Werten verglichen.

Unter den Testbedingungen zelgte Isobutylangelat eine doslsabhängige antiinflammatorische Wirkung am Carrageenin-Induzlerten Rattenpfotenödem, beginnend bei 100 mg/kg. Der ED₅₀ wurde zu 2311 mg/kg Körpergewicht errechnet. Der ED₅₀-Wert für (-)-alpha-Bisabolol wurde mit 1543 mg/kg Körpergewicht bestimmt. Dieser Wert korrespondiert gut mit dem nachstehend aufgeführten Literaturwert.

Im Vergleich hler die Literaturwerte aus :
V. Jakoviev et al. in Journal of Medicinal Plant Research 35 (1979), 125-140.

| Substanz | ED₅₀ (mg/kg) |
|---|---|
| (-) alpha Bisabolol | 1465 |
| (+) alpha Bisabolol | > 2000 |
| Dragosantol | > 3000 |
| Bisabololoxid A | 3164 |
| Bisabololoxid B | > 2000 |
| Bisabolonoxid | > 2000 |

Der Vergleich zeigt, dass von den hier untersuchten Bisabolol-Typen lediglich (-)-alpha-Bisabolol höhere Wirksamkeit aufweist. Insbesondere das weithin Verwendete synthetische Bisabolol "Dragosantol" ist deutlich weniger wirksam als Isobutylangelat. Zudem kommt es als Racemat Im Gegensatz zu Isobutylangelat In der Natur nicht vor.

Nachfolgend werden einige Beispiele für antiphlogistische Zubereltungen unter Verwendung der erfindungsgemäßen Ester angegeben:

### Beispiel 1:

### W/O-Hautcreme

### Zusammensetzung (Angaben in Gew.-%)

| | |
|---|---|
| C12-15 Alkylbenzoat | 9 |
| Glycerin | 7 |
| Polyglyceryl-3-Diisostearat | 6 |
| Caprylsäure Triglycerid | 6 |
| Octyldodecanol | 5 |
| Cyclomethicon | 3 |
| Ethylhexylmethoxyclnnamat | 3 |
| Cetylalkohol | 3 |
| Polyglyceryl-2-Dipolyhydroxystearat | 3 |
| Milchsäure | 2 |
| Isobutylangelat | 0,5 |
| Paraben | 0,1 |
| Wasser dest. | 52,4 |

### Beispiel 2:

### Conditioner Shampoo

### Zusammensetzung (Angaben in Gew.-%)

| | |
|---|---|
| Natriumlaurethsulfat | 10 |
| Cocoamldopropylbetain | 3 |
| Polyquatemium-10 | 0,7 |
| 2-Ethylhexyl Methoxyzinnamat | 0,5 |
| Isoamylangelat | 0,4 |
| Wasser dest. | 85,4 |

### Beispiel 3:

### Sonnenschutzcreme

### Zusammensetzung (Angaben in Gew.-%)

| | |
|---|---|
| Bienenwachs | 10 |
| Ceteareth-20 | 5 |
| Titanium Dioxide (Nanopigment) | 5 |
| Sorbitol (70 %) | 4,5 |
| 3-(4-Methylbenryliden)campher | 4 |
| Dimethicone | 4 |
| PEG-20 Glycerylstearat | 3,5 |
| Mineralöl | 3,5 |
| Dimethicon | 3,5 |
| Isopropyl Lanolat | 3 |
| Behenyl Isostearat | 3 |
| Hexylangelat | 1 |
| Parabene | 0,2 |
| Wasser dest. | 49,8 |

### Beispiel 4:

### After Sun Lotion

### Zusammensetzung (Angaben in Gew.-%)

| | |
|---|---|
| Isostearylisostearat | 15 |
| C10-12 Isoparaffine | 5 |
| PEG-20 Glycerylstearat | 3 |
| Cyclomethicone | 3 |
| Cetearyl Alkohol | 3 |
| Calendula (Extrakt) | 2 |
| Citronellylangelat | 1,5 |
| Parfum | 0,3 |
| Wasser dest. | 67,2 |

### Beispiel 5:

### Calendula Baby-Crème (zur Selbstzubereitung)

### Zusammensetzung (Angaben in Gew.-%)

| | |
|---|---|
| Calendulaöl | 20 |
| Emulsan II | 6 |
| Cetylalkohol | 6 |
| D-Panthenol | 2 |
| Isobutylangelat | 0,5 |
| Wasser dest. | 65,5 |

### Beispiel 6:

### Pflegender Lippenstift

### Zusammensetzung (Angaben in Gew.-%)

| | |
|---|---|
| Ricinusöl | 38 |
| Bienenwachs | 14 |
| Mineralöl | 10 |
| Isopropylpalmitat | 10 |
| Candellilawachs | 8 |
| Hydrierte Kokosfett-Glyceride | 5 |
| Phenyldimethicon | 5 |
| Petrolat | 5 |
| 3-Benzophenon | 4 |
| Isobutylangelat | 1 |

### Beispiel 7:

### Hautschützendes Puder

### Zusammensetzung (Angaben in Gew.-%)

| | |
|---|---|
| Taikum | 84,5 |
| β-Cyclodextrinkomplex mit 9,6 % lsobutylangelat | 8 |
| Bornitrid | 5 |
| Dimethicone | 2 |
| Glycerin | 0,5 |

## Patentansprüche

1. Verwendung von Estern der Angelicasäure zur Anwendung als antiphloglatischen Wirkstoff zur Zubereitung von Mitteln für innerliche und äußerliche Anwendungen, wobei die Veresterung mit Alkoholen erfolgt, ausgewählt aus Methanol, Ethanol, Propanol, Isopropanol, Butanol, isobutanol, sek. Butanol, Amylalkohol, Isoamylalkohol, Neopentylalkohol, 2-Methylbutanol, 3-Mathylbutanol; verzweigten und unverzweigten Hexanolen, Heptanolen, Octanolen, Nonanolen, Decanolen; Allyialkohol, Crotylolkohol, Prenol, Tiglinalkohol, Angellcaalkohol, Senecioalkohol, trans-2-Hexenol, cis-3-Hexenol, Cltronellol, Geraniol, Laurylalkohol, Cetylalkohol, Nerolldol,Phytol, Ethylenglycol, Propylenglycol, Neopentylglycol, Glycerin und monocyclischen Alkoholen.

2. Verwendung nach Anspruch 1, **dadurch gekennzeichnet, dass** die Alkohole ausgewählt sind aus Phenol, Benzylalkohol, Phenylethylalkohol oder Phenylpropylalkohol.

3. Verwendung nach Anspruch 1, **dadurch gekennzeichnet, dass** der Ester lsobutylangelat ist.

4. Verwendung nach einem der Ansprüche 1 bis 3, **dadurch gekennzeichnet, dass** die Einsstzmengen des Esters oder Estergemisches 0,1 bis 0,5 Gew.-% betragen.

5. Verwendung nach einem der Ansprüche 1 bis 4 in Form von Trinkkuren, Kapsein oder Aufgüssen oder mit Trägern ausgewählt aus Salben, Öl-In-Wasser-Emulsionen, Wasser-in-Öl-Emulsionen, Mikroemulsionen und Gelen, oder für topische Anwendungen mit Trägen In Form von Stiftpräparaten, Sprays oder Pudern.

6. Ester der Angelicasäure zur Verwendung als antiphlogistischen Wirkstoff zur Zubereltung von Mitteln für innerliche und äußerliche Anwendungen, wobei die Veresterung mit Alkoholen erfolgt ausgewählt aus Methanol, Ethanol, Propanol, Isopropanol, Butanol, Isobutanol, sek. Butanol, Amylalkohol, Isoamylalkohol, Neopentylalkohol, 2-Methylbutanoi, 3-Methylbutanol; verzweigten und unverzweigten Hexanolen, Heptanolen, Octanolen, Nonanolen, Decanolen; Allylalkohol, Crotylalkohol, Prenol, Tigilnalkohol, Angelicaalkohol, Senecioalkohol, trans-2-Hexenol, cis-3-Hexenol, Cltronellol, Geraniol, Laurylalkohol, Cetylalkohol, Nerolidol, Phytol, Ethylenglycol. Propylenglycol, Neopentyigtycol, Glycerin und monocycilschen Alkoholen.

7. Ester nach Anspruch 6, **dadurch gekennzeichnet, dass** die Alkohole ausgewählt sind aus Phenol, Benzylalkohol, Phenylethylalkohol oder Phenylpropylalkohol.

8. Ester nach Anspruch 6, **dadurch gekennzeichnet, dass** der Ester Isobutylangelat ist

9. Ester nach einem der Ansprüche 6 bis 8, **dadurch gekennzeichnet, dass** die Einsatzmengen des Esters oder Estergemisches 0,1 bis 0,5 Gew.-% betragen.

10. Ester nach einem der Ansprüche 6 bis 8 zur Verwendung in Form von Trinkkuren, Kapseln oder Aufgüssen oder mit Trägern ausgewählt aus Salben, Öl-in-Wasser-Emuisionen, Wasser-in-Öl-Emulsionen, Mikroemulsionen und Gelen, oder für topische Anwendungen mit Trägern In Form von Stiftpräparaten, Sprays oder Pudern.

11. Antiphloglstioche Zubereitung enthaltend eine therapeutisch wirksame Menge eines oder mehrerer Ester der Angelicasäure, erhalten mit Alkoholen ausgewählt aus Methanol, Ethanol, Heptanolen, Ortanolen, Nonanolen, Decanolen; Allylalkohol, Crotylalkohol, Prenol, Tiglinalkohol, Angelicaalkohol, Senecloalkohol, trans-2-Hexenol, cis-3-Hexenol, Citronellol, Geraniol, Laurylalkohol, Cetylalkohol, Nerolldol oder Phytol, Ethylenglycol, Propylenglycol, Neopentylglycol, Glycerin, Laurylaikohol, Cetylalkohol, Nerolidol, Phenol, Benzylalkohol, Phenylethylalkohol und Phenylpropylalkohol.

12. Antiphlogistische Zubereltung nach Anspruch 11, **dadurch gekennzeichnet, dass** diese als Ester Isobutylangelat enthält.

13. Antiphlogistische Zubereitung nach Anspruch 11 oder 12, **dadurch gekennzeichnet, dass** als bezogen auf die Zubereitung 0,1 bis 0,5 Gew.-% einen oder mehrerer Ester der Angelicasäure enthält.

## Claims

1. Use of angelic acid esters for the utilization as antiphlogistic ingredient for the preparation of remedies for Internal and external applications, whereas the esterification Is carried out with alcohols selected from methanol, ethanol, propanol, isopropanol, butanol, isobutanol, sec, butanol, amyl alcohol, Isoamyl alcohol, neopentyl alcohol, 2-methylbutanol, 3-methylbutanol; branched and non-branched hexanols, heptanols, octanols, nonanols, decanols; allyl alcohol, crotyl alcohol, prenol, tiglin alcohol, angelic alcohol, seneclo alcohol, trans-2-hexenol, cis-3-hexenol, citronellol, geraniol, lauryl alcohol, cetyl alcohol, nerolidol, phytol, ethylene glycol, propylene glycol, neopentyl glycol, glycerlne and monocyclic alcohols.

2. Use according to claim 1, **characterized in that** the alcohols are chosen from phenol, benzyl alcohol, phenyl ethyl alcohol or phenyl propyl alcohol.

3. Use according to claim 1, **characterized in that** the ester is isobutyl angelate.

4. Use according to one of the claims 1 to 3, **characterized in that** the ester or ester mixture is Included In an amount of 0.1 to 0.6 percent per weight.

5. Use according to one of the claims 1 to 4, in form of drinking cures, capsules or Infusions or with carriers selected from salves, oil-in-water emulsions, water-in-oil emulsions, micro emulsions and gels, or for topical applications with carriers In form of stick preparations, sprays or powders.

6. Esters of angelic acid for use as antiphiogistic ingredient for the preparation of remedies for internal and external applications, whereas the esterification is carried out with alcohols selected from methanol, ethanol, propanol, lsopropanol, butanol, lsobutanol, sec. butanol, amyl alcohol, Isoamyl alcohol, neopentyl alcohol, 2-mothylbutanol, 3-methylbutanol; branched and non-branched hexanols, heptanols, octanols, nonanols, decanols; allyl alcohol, crotyl alcohol, prenol, tiglin alcohol, angelic alcohol, senecio alcohol, trans-2-hexenol, cls-3-hexenol, citronellol, geraniol, lauryl alcohol, cetyl alcohol, nerolidol, phytol, ethylene glycol, propylene glycol, neopentyl glycol, glycerine and monocyclic alcohols.

7. Ester according to claim 6, **characterized in that** alcohols are chosen from phenol, benzyl alcohol, phenyl ethyl alcohol or phenyl propyl alcohol.

8. Ester according to claim 6, **characterized in that** the ester Is Isobutyl angelate.

9. Ester according to one of the claims 6 to 8, **characterized in that** the ester or ester mixture Is Included in an amount of 0.1 to 0.5 percent per weight.

10. Ester according to one of the claims 6 to 9 for use in form of drinking cures, capsules or infusions or with carriers selected from salves, oil-in-water emulsions, water-In-oil emulsions, micro emulsions and gels, or for topical application with carriers In form of stick preparations, sprays or powders.

11. Antiphlogistic preparation comprising a therapeutic effective dose of one or several ester of angelic acid, obtained with alcohols selected from methanol, ethanol, heptanols, octanols, nonanols, decanois; allyl alcohol, crotyl alcohol, prenol, tiglin alcohol, angelic alcohol, senecio alcohol, trans-2-hexenol, cis-3-hexenol, citronellol, geraniol, lauryl alcohol, cetyl alcohol, nerolidol or phytol, ethylene glycol, propylene glycol, neopentyl glycol, glycerine, lauryl alcohol, cetyl alcohol, nerolidol, phenol, benzyl alcohol, phenyl ethyl alcohol and phenyl propyl alcohol.

12. Antiphlogistic preparation according to claim 11, **characterized by** comprising Isobutyl angelate as ester.

13. Antiphlogistic preparation according to claim 11 or 12, **characterized by** comprising one or several ester of angelic acid In an amount of 0.1 to 0.5 percent per weight referring to the preparation.

## Revendications

1. Utilisation d'esters d'acide angélique pour leur application sous forme d'agent antiphloglatique pour la préparation d'agents pour applications Internes et externes, l'estérification ayant lieu avec des alcools choisis parmi le méthanol, éthanol, propanol, Isopropanol, butanol, Isobutanol, butanol secondaire, alcool amylique, alcool isnamylique, alcool néopentylique, 2-méthylbutanol, 3-méthylbutanol ; hexanols, heptanols, octanols, nonanols, décanols ramifiés et non ramifiés ; alcool allylique, alcool crotylique, prénol, alcool tiglinique, alcool angélique, alcool de Seneclo, trans-2-hexenol, cls-3-hexenol, huile de citronnelle, géraniol, alcool laurique, alcool cétylique, nérolldol, phytol, êthylèneglycol, propylèneglycol, néopentylglycol, glycérine et des alcools monocycliques.

2. Utilisation selon la revendication 1, **caractérisée en ce que** les alcools sont choisis parmi le phénol, l'alcool benzylique, l'alcool phényléthylique ou l'alcool phénylpropylique.

3. Utilisation selon la revendication 1, **caractérisée en ce que** l'ester est de l'angelate d'isobutyle.

4. Utilisation selon l'une quelconque des revendications 1 à 3, **caractérisée en ce que** les quantités utilisées d'ester ou de mélange d'ester sont comprises entre 0,1 et 0,5 % en poids.

5. Utilisation selon l'une quelconque des revendications 1 à 4 sous forme de diètes hydriques, de capsules ou d'infusions ou à l'aide de supports choisis parmi les pommades, les émulsions d'huile dans l'eau, les émulsions d'eau dans l'huile, les microémulsions et les gels, ou pour des applications topiques à l'aide de supports se présentant sous forme de préparations en bâton, de sprays ou de poudres.

6. Ester d'acide angélique destiné à être utilisé sous forme d'agent antiphlogistique pour la préparation d'agents pour applications internes et externes, l'estérification ayant lieu avec des alcools choisis parmi le méthanol, éthanol, propanol, lsopropanol, butanol, Isobutanol, butanol secondaire, alcool amylique, alcool isoamylique, alcool néopentylique, 2-méthylbutanol, 3-méthylbutanol ; hexanols, heptanols, octanols, nonanols, décanols ramifiés et non ramifiés ; alcool allylique, alcool crotyllque, prénol, alcool tiglinique, alcool angélique, alcool de Seneclo, trans-2-hexenol, cls-3-hexenol, huile de citronnelle, géraniol, alcool laurique, alcool cétylique, nérolidol, phytol, éthylèneglycol, propylèneglycol, néopentylglycol, glycérine et des alcools monocycliques.

7. Ester selon la revendication 6, **caractérisé en ce que** les alcools sont choisis parmi le phénol, l'alcool benzylique, l'alcool phényléthylique ou l'alcool phénylpropylique.

8. Ester selon la revendication 6, **caractérisé en ce que** l'ester est de l'angelate d'isobutyle.

9. Ester selon l'une quelconque des revendications 6 à 8, **caractérisé en ce que** les quantités utilisées d'ester ou de mélange d'ester sont comprises entre 0,1 et 0,5 % en poids.

10. Ester selon l'une quelconque des revendications 6 à 9 destiné à être utilisé sous forme de diètes hydriques, de capsules ou d'infusions ou à l'aide de supports choisis parmi les pommades, les émulsions d'huile dans l'eau, les émulsions d'eau dans l'huile, les microémulsions et les gels, ou pour des applications topiques à l'aide de supports se présentant sous forme de préparations en bâton, de sprays ou de poudres.

11. Préparation antiphlogistique comprenant une quantité à effet thérapeutique d'un ou de plusieurs esters d'acide angélique, obtenue à l'aide d'alcools choisis parmi le méthanol, l'éthanol, les heptanols, octanols, nonanols, décanols ; alcool allylique, alcool orotylique, prénol, alcool tiglinique, alcool angélique, alcool de Senecio, trans-2-hexenol, cis-3-hexenol, huile de citronnelle, géranlol, alcool laurique, alcool cétylique, nérolidol ou phytol, éthylèneglycol, propylèneglycol, néopentylglycol, glycérine, alcool laurique, alcool cétylique, nérolldol, phénol, alcool benzylique, alcool phényléthylique ou alcool phénylpropylique.

12. Préparation antiphlogistique selon la revendication 11, **caractérisée en ce que** ceile-cl contient comme ester de l'angelate d'isobutyle.

13. Préparation antiphlogistique selon la revendication 11 ou 12, **caractérisée en ce qu'**en ce qui concerne la préparation, elle comprend entre 0,1 et 0,5 % en poids d'un ou de plusieurs esters d'acide angélique.
